# EUROPÄISCHE PATENTSCHRIFT

(11) **EP 2 062 876 B1**
(45) Veröffentlichungstag und Bekanntmachung des Hinweises auf die Patenterteilung: **27.07.2011**
(21) Anmeldenummer: 08019800.5
(22) Anmeldetag: 13.11.2008
(51) Int. Cl.: C07C 263/10, C07C 265/14

(54) **Verfahren zur Herstellung aromatischer Diisocyanate in der Gasphase**
Method for manufacturing aromatic diisocyanates in the gaseous phase
Procédé destiné à la fabrication de diisocyanates aromatiques en phase gazeuse

(30) Priorität: 22.11.2007 DE 102007056511
(43) Veröffentlichungstag der Anmeldung: 27.05.2009
(73) Patentinhaber: Bayer MaterialScience AG, 51368 Leverkusen (DE)
(72) Erfinder: Pohl, Fritz, Dr., 25541 Brunsbüttel (DE); Biskup, Klaus, Dr., 51373 Leverkusen (DE); Bruns, Rainer, Dr., 51373 Leverkusen (DE); Steffens, Friedhelm, 51373 Leverkusen (DE); Padeken, Lars, Dr., 40223 Düsseldorf (DE)

(56) Entgegenhaltungen:
- EP-A- 0 570 799
- EP-A- 1 935 876
- US-A- 5 391 683

## Beschreibung

Die Erfindung betrifft ein Verfahren zur Herstellung primärer aromatischer Diisocyanate durch Umsetzung der entsprechenden primären aromatischen Diamine mit Phosgen in der Gasphase, bei dem Phosgen und die primären aromatischen Diamine innerhalb einer mittleren Verweilzeit von 0,05 bis 15 Sekunden umgesetzt werden und die eingesetzten primären aromatischen Diamine weniger als 0,05 mol-% an keine Ketogruppen enthaltenden, aliphatischen Amine in der Summe pro mol primärer Aminogruppen enthalten.

Es sind aus dem Stand der Technik verschiedene Verfahren zur Herstellung von Diisocyanaten durch Umsetzung von Diaminen mit Phosgen in der Gasphase bekannt, wobei speziell die Phosgenierung von aliphatischen Diaminen in der Gasphase bereits hinlänglich beschrieben ist.

So offenbart EP-B1-0 289 840 ein Verfahren zur Herstellung von Diisocyanaten der allgemeinen Formel OCN-R-NCO, in welcher R für einen (cyclo)-aliphatischen Kohlenwasserstoffrest mit bis zu 15 Kohlenstoffatomen steht, durch Phosgenierung der entsprechenden Diamine der allgemeinen Formel H₂N-R-NH₂ in der Gasphase, dadurch gekennzeichnet, dass man die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und kontinuierlich in einem auf 200 bis 600 °C erhitzten, zylindrischen Reaktionsraum ohne sich bewegende Teile unter Aufrechterhaltung einer turbulenten Strömung im Reaktionsraum miteinander zur Reaktion bringt, das den Reaktionsraum verlassende Gasgemisch durch ein inertes Lösungsmittel leitet, welches bei einer Temperatur oberhalb der Zersetzungstemperatur des dem Diamin entsprechenden Carbamidsäurechlorids gehalten wird, und das in dem inerten Lösungsmittel gelöst vorliegende Diisocyanat einer destillativen Aufarbeitung unterzieht.

Das in EP-B1-0 289 840 beschriebene Verfahren ist in weiteren Veröffentlichungen mehrfach sowohl im Hinblick auf seine Anwendungsbreite wie auch spezieller apparativer Lösungen modifiziert worden. So offenbart EP-B1-0 749 958 die Umsetzung (cyclo)aliphatischer Triamine mit drei primären Aminogruppen in der Gasphase in einem Rohrreaktor bei 200 bis 600 °C, EP-B1-1078 918 die Anwendung der Basisprinzipien von EP-B1-0 289 840 auf die Gasphasenphosgenierung von (cyclo)aliphatischen Diaminen mit zwei primären Aminogruppen in 1,2- oder 1,3-Stellung zueinander. EP-B1-1 275 639 B1 und EP-B1-1 275 640 offenbaren spezielle Reaktorkonfigurationen einer verbesserten Durchmischung der in den Reaktionsraum eingespeisten Reaktanden.

Auch die Umsetzung aromatischer Diamine mit Phosgen in der Gasphase zu den entsprechenden Diisocyanaten ist in der Literatur beschrieben.

EP-B1- 0 593 334 offenbart ein Verfahren zur Herstellung von aromatischen Diisocyanaten in der Gasphase, wobei ein Rohrreaktor verwendet wird. Darin wird eine Vermischung der Edukte ohne bewegliches Rühren durch eine Einengung der Wände erreicht. Die Umsetzung erfolgt im Temperaturbereich von 250 bis 500°C. Das Verfahren ist jedoch problematisch, da die Vermischung der Eduktströme allein über eine Verengung der Rohrwand schlecht im Vergleich zur Anwendung eines richtigen Mischorgans funktioniert, eine schlechte Vermischung üblicherweise zu einer unerwünscht hohen Feststoffbildung führt.

EP-A-0 699 657 offenbart entsprechend ein Verfahren zur Herstellung von aromatischen Diisocyanaten in der Gasphase in einem Mischreaktor, wobei der Mischreaktor in zwei Zonen aufgeteilt wird, wovon die erste eine vollkommene Durchmischung der Edukte und die zweite eine kolbenartige Strömung gewährleistet. Doch auch bei diesem Verfahren treten Probleme durch Bildung von Feststoffen auf, welche die Misch- und Reaktionsvorrichtungen verstopfen und die Ausbeute vermindern.

Es hat nicht an Versuchen gefehlt, die insbesondere bei der Umsetzung von aromatischen Diaminen mit Phosgen in der Gasphase zu beobachtende Bildung von Feststoffen zu minimieren um so eine großtechnisch durchführbare Phosgenierung von aromatischen Diaminen in der Gasphase zu ermöglichen.

EP-B1- 0 570 799 offenbart ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Verweilzeit von 0,5 bis 5 Sekunden durchgeführt wird. Nach der Lehre von EP-B1- 0 570 799 ist zur Minimierung der unerwünschten Feststoffbildung eine Vergleichmäßigung der Strömung in dem Reaktionsraum erforderlich und vor allem eine Rückvermischung der Komponenten in dem Reaktionsraum auszuschließen. Es wird daher ein Verfahren offenbart, bei dem die mittlere Abweichung von der mittleren Verweilzeit weniger als 6% beträgt. Die Einhaltung dieser Verweilzeit wird dadurch erreicht, dass die Reaktion in einer Rohrströmung durchgeführt wird, die entweder durch eine Reynolds-Zahl von oberhalb 4.000 oder einer Bodenstein-Zahl von oberhalb 100 charakterisiert wird.

Maßnahmen zur Vergleichmäßigung der Strömungsverhältnisse sind ebenfalls Gegenstand von EP-B1-1 362 847. EP-B1-1 362 847 offenbart ein Verfahren zur Herstellung von aromatischen Disocyanaten in der Gasphase, bei dem durch eine verbesserte Reaktionsführung in Rohrreaktoren durch strömungstechnische Maßnahmen wie der Vergleichmäßigung und Zentrierung der Eduktströme zeitliche Schwankungen der Temperatur und eine Asymmetrie in der Temperaturverteilung vermieden werden, die nach der Lehre von EP-B1-1 362 847 zu Anbackungen und Verstopfungen im Reaktor und damit zur Verkürzung der Standzeit der Reaktoren führen.

Nach der Lehre von EP-A1-1 449 826 steht bei der Umsetzung der aromatischen Diamine mit Phosgen in der Gasphase die Umsetzung des Phosgens mit dem Diamin zu dem Diisocyanat in Konkurrenz zu der Folgereaktion des Diamins mit dem Diisocyanat zum entsprechenden Harnstoffoligomer, wobei eine verbesserte Mischung der Edukte Phosgen und Diamin bei gleichzeitiger Vermeidung von Rückströmung durch Wirbel im Rohrreaktor die Selektivität der Diisocyanatbildung erhöht und die Harnstoffbildung reduziert. Dadurch lassen sich nach der Lehre von EP-A1-1 449 826 die Mengen an Kondensationsprodukt im Rohrreaktor verringern, die aufgrund ihrer Ablagerung an der Reaktorwand zu einer Verkleinerung des freien Rohrquerschnitts und zu allmählichem Druckanstieg im Reaktor führen und letztlich die Standzeit des Verfahrens bestimmen. Apparative Lösungen zur verbesserten Mischung der Edukte offenbaren ebenfalls EP-A1-1 526 129, DE-A-103 59 627 und WO 2007/028 751 A, wobei strömungstechnische Maßnahmen zur Drallerzeugung (EP-A1-1 526 129), konzentrisch angeordnete Ringspaltdüsen mit singulärer (DE-A-103 59 627) oder mehrfacher Amineinspeisung (WO 2007/028 751 A) wie auch mehrere, parallel zur Achse eines Rohrreaktors angeordnete Amindüsen (EP-A1-1 449 826) zum Einsatz kommen.

Ausführlich behandelt WO-A-2003/045 900 die Frage der Temperaturführung bei der Herstellung von Isocyanaten im großtechnischen Maßstab unter Anwendung einer Gasphasenphosgenierung. Wie in WO-A-2003/045 900 dargestellt, bieten sich bei den bekannten Verfahren zur Gasphasenphosgenierung, die einen zylindrischen Reaktionsraum verwenden, zwei Möglichkeiten der technischen Realisierung an. Zum einen kann die Reaktion in einer einzigen Rohrstrecke durchgeführt werden, deren Durchmesser an die Produktionskapazität der Anlage angepasst werden muss. Gemäß WO-A-2003/045 900 hat dieses Konzept für sehr große Produktionsanlagen den Nachteil, dass eine genaue Temperierung der Reaktionsströme im Kern der Strömung durch eine Wandheizung /-kühlung des Rohres nicht mehr realisierbar ist, lokale Temperatur-Inhomogenitäten aber bei zu hoher Temperatur zur Produktzersetzung, bei zu niedriger Temperatur zur ungenügenden Umsetzung der Edukte zum gewünschten Isocyanat führen können. Auch die zweite Möglichkeit der technischen Realisierung, die Aufteilung des reagierenden Gemisches in einzelne Teilströme, die dann parallel durch kleinere, einzelne Rohre geleitet werden, die aufgrund ihres kleineren Durchmessers besser temperierbar sind, wird von WO-A-2003/045900 als nachteilig angesehen. Gemäß WO-A-2003/045900 ist bei dieser Verfahrensvariante nachteilig, dass diese Variante relativ verstopfungsanfällig ist, wenn nicht der Volumenstrom durch jedes einzelne Rohr geregelt wird. Nach der Lehre von WO2003/045 900 können die skizzierten Nachteile dadurch umgangen werden, dass die kontinuierliche Phosgenierung von Aminen in der Gasphase vorteilhaft, d.h. beispielsweise bei wesentlich erhöhter Betriebsstundenzahl der Produktionsanlage, durchgeführt werden kann, wenn die Reaktion in einem nicht zylindrischen Reaktionskanal, bevorzugt einem Plattenreaktor, durchgeführt wird, der bevorzugt eine Höhe aufweist, welche eine vorteilhafte Temperierung der Reaktanden ermöglicht und der eine Breite aufweist, die mindestens das Zweifache der Höhe beträgt. Wie WO-A-2003/045 900 weiter ausführt, ist die Höhe des Reaktionskanals im Allgemeinen nicht beschränkt und kann die Umsetzung in einem Reaktionskanal mit einer Höhe von beispielsweise 40 cm erfolgen. Es soll jedoch ein besserer Wärmeaustausch mit den Reaktorwänden erfolgen. Daher lehrt WO-A-2003/045 900 die Umsetzung in Reaktionskanälen mit geringer Höhe.

Nach der Lehre von WO2007-A-028 715 müssen Amine, die in einer Gasphasenphosgenierung zu den entsprechenden Isocyanaten umgesetzt werden können, bestimmte Erfordernisse erfüllen. Danach sind speziell solche Amine geeignet, die ohne signifikante Zersetzung in die Gasphase überführt werden können. Nach der Lehre von WO2007-A-028 715 sind dies Amine, die sich während der Dauer der Reaktion unter den Reaktionsbedingungen zu höchstens 2 mol%, besonders bevorzugt zu höchstens 1 mol% und ganz besonders bevorzugt zu höchstens 0,5 mol% zersetzen.

Nach der Lehre von EP-A-1 754 689 ist die partielle Zersetzung unter Ammoniak-Abspaltung der in die Gasphasenphosgenierung eingesetzten Amine nicht allein während der Reaktion zu beachten sondern ist diese ebenfalls bei der Verdampfung der Amine zu berücksichtigen. Die partielle Zersetzung unter Ammoniak-Abspaltung während der Verdampfung vermindert nicht nur die Ausbeute, sondern es bilden sich bei der nachfolgenden Phosgenierungsreaktion auch Ablagerungen von Ammoniumchlorid in den nach geschalteten Rohrleitungen und Apparaten. Die Anlagen müssen dann relativ häufig gereinigt werden, wobei entsprechende Produktionsverluste entstehen.

Trotz der Versuche, die Umsetzung von aromatischen Aminen mit Phosgen in der Gasphase zu optimieren und dabei insbesondere die bei der Umsetzung von aromatischen Diaminen mit Phosgen in der Gasphase zu beobachtende Bildung von Feststoffen zu minimieren, besteht ein weiterer Bedarf zur Verbesserung der Gasphasenphosgenierung aromatischer Diamine, um so eine großtechnisch durchführbare Phosgenierung von aromatischen Diaminen in der Gasphase zu ermöglichen.

Aufgabe der Erfindung war es daher, ein einfaches und wirtschaftliches Verfahren zur Herstellung von aromatischen Diisocyanaten aus den entsprechenden aromatischen Diaminen durch Phosgenierung in der Gasphase bereitzustellen, das eine technisch vorteilhafte Umsetzung, insbesondere im Hinblick auf eine hohe Raum-Zeit-Ausbeute und ein geringes Auftreten von störenden Feststoffen und den damit verbundenen Anbackungen und Verstopfungen gewährleistet.

Diese Aufgabe konnte überraschend dadurch gelöst werden, dass Phosgen und die primären aromatischen Diamine innerhalb einer mittleren, hydrodynamischen Verweilzeit von 0,05 bis 15 Sekunden umgesetzt werden und die eingesetzten primären aromatischen Diamine weniger als 0,05 mol-% an keine Ketogruppen enthaltenden, aliphatischen Amine in der Summe pro mol primärer Aminogruppen enthalten. Denn die aliphatischen, keine Ketogruppen enthaltenden Amine sind maßgeblich für die Bildung von Feststoffablagerungen in der Gasphasenphosgenierung verantwortlich.

Gegenstand der Erfindung ist daher ein Verfahren zur Herstellung primärer aromatischer Diisocyanate durch Umsetzung der entsprechenden primären aromatischen Diamine mit Phosgen in der Gasphase, dadurch gekennzeichnet, dass
a) Phosgen und die primären aromatischen Diamine innerhalb einer mittleren Verweilzeit von 0,05 bis 15 Sekunden umgesetzt werden und
b) die eingesetzten primären aromatischen Diamine weniger als 0,05 mol-%, bevorzugt weniger als 0,04 mol-%, besonders bevorzugt 0,001 bis 0,035 mol-% an keine Ketogruppen enthaltenden, aliphatischen Amine in der Summe pro mol primärer Aminogruppen enthalten.

Unter den keine Ketogruppen enthaltenden, aliphatischen Aminen sind sowohl lineare als auch verzweigte aliphatische Amine wie auch cycloaliphatische Amine zu verstehen, die ggf. auch substituiert oder ein- oder mehrfach ungesättigt sein können, solange sie keine Ketogruppen aufweisen. Typische Substituenten sind beispielsweise Nitro-, Nitroso- oder Cl-Substituenten.

Weiterhin umfassen die keine Ketogruppen enthaltenden, aliphatischen Aminen auch mehrkernige oder verbrückte cycloaliphatische Amine, die ggf. auch substituiert oder ein- oder mehrfach ungesättigt sein können, solange sie keine Ketogruppen aufweisen. Die mehrkernigen oder verbrückten cycloaliphatischen Amine können direkt verbunden oder über ein oder mehrere Heteroatome, insbesondere über ein- oder mehrere Stickstoffatome verbrückt sein.

Weiterhin sind unter den molen primärer Aminogruppen die Summe der Aminogruppen aus den primären aromatischen Aminen und aus den keine Ketogruppen enthaltenden, aliphatischen Aminen zu verstehen.

Der erfindungsgemäß notwendige niedrige Gehalt an keine Ketogruppen enthaltenden aliphatischen Aminen in den in der angestrebten Gasphasenphosgenierung umzusetzenden aromatischen Diaminen ist insofern überraschend, als die gleichzeitige Phosgenierung aliphatischer und aromatischer primärer Aminogruppen grundsätzlich dem Stand der Technik entspricht (DE-A 2249459, DE-A- 198 28 510) In der Literatur wird zwar die Stabilität der in eine Gasphasenphosgenierung eingesetzten Amine im Hinblick auf ihre Zersetzung unter Desaminierung behandelt, jedoch können aus den angegebenen Verdampfungs- und Reaktionsbedingungen keine Angaben zur notwendigen relativen Stabilität der gebildeten Isocyanate bei dem gleichzeitigen Einsatz aromatischer und (cyclo)aliphatischer primärer Amingruppen hergeleitet werden. Insbesondere ergeben sich aus dem Stand der Technik keine Hinweise, dass insbesondere die keine Ketogruppen enthaltenden aliphatischen Amine problematisch sind.

Das erfindungsgemäße Verfahren ist zwar grundsätzlich zur Gasphasenphosgenierung aller nach dem Stand der Technik als unter den Reaktionsbedingungen stabil zu bezeichnenden (WO-A-2007/0287515) aromatischen Diamine (z.B. Naphthalindiamine (NDA), insbesondere 1,5-NDA und 1,8-NDA, Diamine der Diphenylmethanreihe (MDA), insbesondere 2,2'-MDA, 2,4'-MDA und 4,4'-MDA sowie Toluylendiamin (TDA), insbesondere 2,4-TDA und 2,6-TDA) geeignet, findet bevorzugt jedoch Anwendung bei der Umsetzung von Toluylendiamin als Isomerengemisch mit Phosgen in der Gasphase zu den entsprechenden Toluylendiisocyanaten. Besonders bevorzugt ist die Anwendung des Verfahrens bei der Gasphasenphosgenierung von 2,4- / 2,6-Toluylendiamin-Gemischen, ganz besonders bevorzugt von 2,4-/2,6-Toluylendiamin-Gemischen der Isomerenverhältnisse 80/20 und 65/35.

Die bevorzugte Anwendung des erfindungsgemäßen Verfahrens bei der Umsetzung von Toluylendiamin mit Phosgen in der Gasphase ist insofern von besonderer Bedeutung, als die Herstellung von Toluylendiamin im großtechnischen Maßstab speziell durch katalytische Hydrierung von Dinitrotoluolen erfolgt, wobei vorwiegend durch Nitrierung von Toluol mit Salpetersäure nach dem Mischsäureverfahren zugängliche Gemische isomerer Dinitrotoluole eingesetzt werden (Ullmann's Enzyklopedie der technischen Chemie, 4.Auflage, Band 7, Seite 393ff, 1973, Verlag Chemie Weinheim / New York). Bei der Hydrierung finden neben der gewünschten Nitrogruppenreduktion ebenfalls Kernhydrierungen sowie weitere Nebenreaktionen zu aliphatischen oder cycloaliphatischen oder mehrkernigen oder verbrückten Nebenprodukten mit primärer Amingruppen statt.

Bevorzugt weist das erfindungsgemäßen Verfahren einen oder mehrere der folgenden Schritte a) - d) auf, wobei besonders bevorzugt alle Schritte a) - d) durchgeführt werden: Die Schritte a) - d) sind dadurch gekennzeichnet, dass man
a) die dampfförmigen aromatischen Diamine, gegebenenfalls verdünnt mit einem Inertgas oder mit den Dämpfen eines inerten Lösungsmittels, und Phosgen getrennt auf Temperaturen von 200 bis 600 °C erhitzt und kontinuierlich vermischt, wobei ein Reaktionsgemisch erhalten wird,
b) das in Schritt a) erzeugte Reaktionsgemisch unter Vermeidung von Rückvermischung kontinuierlich durch einen Reaktionsraum leitet und es darin innerhalb einer mittleren Verweilzeit von 0,05 bis 15 Sekunden umsetzt, wobei ein Gasgemisch erhalten wird,
c) das aus dem Reaktionsraum austretende Gasgemisch zur Kondensation der gebildeten aromatischen Diisocyanate abkühlt, wobei die Temperatur oberhalb der Zersetzungstemperatur der den umgesetzten aromatischen Diaminen entsprechenden Carbamidsäurechloriden gehalten wird, und
d) nicht kondensiertes aromatisches Diisocyanat aus dem Gasgemisch durch Waschen mit einer Waschflüssigkeit abtrennt.

Bevorzugt wird in Schritt b) ein Reaktionsraum eingesetzt, der eine rotationssymmetrische Geometrie mit einer in Strömungsrichtung des Reaktionsgemischs konstanten oder steigenden durchströmten Querschnittsfläche aufweist. Bevorzugt wird als Reaktionsraum ein Rohrreaktor mit einer in Strömungsrichtung des Reaktionsgemischs im Wesentlichen konstanten oder steigenden durchströmten Querschnittsfläche eingesetzt. In einer weiteren bevorzugten Ausführungsform weist der Reaktionsraum, bevorzugt ein Rohrreaktor, in Strömungsrichtung Abschnitte konstanter und steigender Querschnittsfläche auf.

Die erfindungsgemäß geeignete Ausführung des Reaktionsraums mit rotationssymmetrischer Geometrie und einer in Strömungsrichtung kaskadenförmigen und/oder kontinuierlichen Veränderung der durchströmten Querschnittsfläche hat den Vorteil, dass die Strömungsgeschwindigkeit entlang der Achse des Reaktionsraums eingestellt werden kann. Eine in Strömungsrichtung konstante durchströmte Querschnittsfläche führt wegen der Volumenvergrößerung während der Phosgenierung zu einer Beschleunigung der Strömung. Durch eine in Strömungsrichtung geeignet gewählte Erweiterung der durchströmten Querschnittsfläche kann die Strömungsgeschwindigkeit der Reaktionsmischung über die Länge des Reaktors konstant gehalten werden.

Bevorzugt wird der Reaktionsraum in Schritt b) nach dem Strahlmischerprinzip mit den gemäß Schritt a) bereiteten und erhitzten Komponenten beschickt. Beim Strahlmischerprinzip (Chemie-Ing.-Techn. 44, (1972) S. 1055, Abb. 10) werden einem rotationssymmetrischen Reaktionsraum zwei Eduktströme A und B zugeführt, wobei der Eduktstrom A über eine Zentraldüse und der Eduktstrom B über einen Ringraum zwischen Zentraldüse und Reaktorwand zugeführt werden. Die Strömungsgeschwindigkeit des Eduktstroms A ist groß gegenüber der Strömungsgeschwindigkeit des Eduktstroms B. Hierdurch kommt es im rotationssymmetrischen Reaktionsraum zu einer Vermischung der Reaktionspartner und in der Folge zu deren Reaktion. Die Durchmischung der Komponenten kann durch strömungstechnische Maßnahmen, wie sie z.B. in EP-B1-1 362 847 und EP-A1-1 526 129 beschrieben sind, verbessert werden, auch können Abwandlungen des Basisprinzips, wie konzentrisch angeordnete Ringspaltdüsen mit singulärer Aminspeisung (DE-A-103 59 627) oder mehrere, parallel zur Achse eines Rohrreaktors angeordnete Amindüsen (EP-A1-1 449 826) zum Einsatz kommen. Es ist ebenfalls möglich, gemäß der Lehre von (WO 2007/028 751 A) die Komponenten über eine konzentrisch angeordnete Ringspaltdüse mit einfacher oder mehrfacher Amineinspeisung in den Reaktionsraum zu speisen. Auf die zitierten Anmeldungen und die dort aufgeführten Maßnahmen zur Verbesserung der Mischung und der dafür notwendigen charakteristischen Kenngrößen wird ausdrücklich Bezug genommen.

Die aromatischen Ausgangsamine werden vor der Durchführung des erfindungsgemäßen Verfahrens in der Regel verdampft und auf 200°C bis 600°C , bevorzugt 225 bis 500°C, besonders bevorzugt 250°C bis 450°C erhitzt und gegebenenfalls verdünnt mit einem Inertgas wie N₂, He, Ar oder mit den Dämpfen eines inerten Lösungsmittels, z.B. aromatischen Kohlenwasserstoffen ggf. mit Halogensubstitution, wie z.B. Chlorbenzol oder o-Dichlorbenzol, dem Reaktionsraum zugeführt.

Die Verdampfung der aromatischen Ausgangsamine kann in allen bekanten Verdampfungsapparaturen erfolgen. Bevorzugt werden Verdampfungssysteme eingesetzt, bei denen ein kleiner Arbeitsinhalt mit einer hohen Umwälzleistung über einen Fallfilmverdampfer geführt wird. Dabei wird bevorzugt zur Minimierung der thermischen Belastung der Ausgangsamine der Verdampfungsvorgang - wie oben ausgeführt- ggf. unter Zuspeisung von Inertgas und / oder Dämpfen eines inerten Lösungsmittels unterstützt. Der Einsatz von Mikrowärmeaustauscher, wie in EP-A-1 754 689 fenbart, ist jedoch ebenfalls möglich. Die dampfförmigen aromatischen Diamine können noch Anteile an unverdampften Tröpfchen an aromatischen Diaminen (Aerosol) enthalten. Bevorzugt enthalten die dampfförmigen aromatischen Diamine jedoch im Wesentlichen keine Tröpfchen an unverdampften aromatischen Diaminen, das heißt maximal 0,5 Mass.-% des aromatischen Diamins, besonders bevorzugt maximal 0,05 Mass.-% des aromatischen Diamins, bezogen auf die Gesamtmasse des aromatischen Diamins, liegt in der Form von unverdampften Tröpfchen vor und der restliche Teil des aromatischen Diamins liegt dampfförmig vor. Ganz besonders bevorzugt enthalten die dampfförmigen aromatischen Diamine keine Tröpfchen an unverdampften aromatischen Diaminen. Bevorzugt wird nach der Verdampfung das dampfförmige aromatische Diamin, ggf. mit Inertgasen oder inerten Lösungsmitteldämpfen verdünnt, über einen Nacherhitzer auf die gewünschte Einsatztemperatur gebracht.

Weiterhin bevorzugt erfolgt die Verdampfung und Überhitzung der Ausgangsamine mehrstufig, um unverdampfte Tröpfchen im dampfförmigen Aminstrom zu vermeiden. Insbesondere bevorzugt sind mehrstufige Verdampfungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z.B. beschrieben in "Droplet Separation", A. Bürkholz, VCH Verlagsgesellschaft, Weinheim - New York - Basel - Cambridge, 1989. Besonders bevorzugt sind Tröpfchenabscheider die einen geringen Druckverlust verursachen. Ganz besonders bevorzugt wird das verdampfte aromatische Diamin über einen Nacherhitzer auf die gewünschte Einsatztemperatur gebracht, der auch als Tröpfchenabscheider fungiert. Insbesondere bevorzugt hat dieser Nacherhitzer einen Flüssigkeitsablauf um die stetige Entleerung des Abscheiders zu gewährleisten. Durch die Erzeugung eines im Wesentlichen Tröpfchen freien dampfförmigen Ausgangsaminstromes vor Eintritt in den Reaktor wird die Reaktorlaufzeit deutlich erhöht.

Bei dem erfindungsgemäßen Verfahren ist es vorteilhaft, Phosgen im Überschuss bezüglich Aminogruppen einzusetzen. Üblicherweise liegt ein molares Verhältnis von Phosgen zu Aminogruppen von 1,1 : 1 bis 20 : 1, bevorzugt 1,2 : 1 bis 5 : 1 vor. Auch das Phosgen wird bevorzugt auf Temperaturen von 200°C bis 600°C erhitzt und gegebenenfalls verdünnt mit einem Inertgas wie N₂, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktionsraum zugeführt. Weiterhin kann das dem Reaktionsraum zugeführte Phosgen mit HCl oder CO verdünnt sein.

Bevorzugt wird das erfindungsgemäße Verfahren so durchgeführt, dass die getrennt aufgeheizte Reaktionspartner in wenigstens einen Reaktionsraum eingeführt, vermischt und unter Beachtung geeigneter Reaktionszeiten unter adiabater Reaktionsführung umgesetzt werden. Anschließend wird durch Abkühlung des Gasstromes das Isocyanat kondensiert, wobei die Abkühlung bis zu einer Temperatur oberhalb der Zersetzungstemperatur des entsprechenden Carbaminsäurechlorids, also beispielsweise Toluylendiaminsäurechlorid im Falle von TDA, erfolgt.

Die notwendige Verweilzeit zur Reaktion der aromatischen Amingruppen mit dem Phosgen zu Isocyanat liegt zwischen 0,05 und 15 Sekunden, bevorzugt zwischen 0,1 und 10 Sekunden, und ist abhängig von der Art des eingesetzten aromatischen Diamins, der Start-Reaktionstemperatur, der adiabaten Temperaturerhöhung im Reaktionsraum, dem molaren Verhältnis von eingesetztem aromatischen Diamin bzw. Aminogruppen und Phosgen, dem Druck im Reaktionsraum und einer etwaigen Verdünnung der Reaktionspartner mit Inertgasen.

Wird für das jeweilige System (Start-Reaktionstemperatur, adiabate Temperaturerhöhung, molares Verhältnis der Reaktanden, Verdünnungsgas, eingesetztes aromatisches Diamin, Druck im Reaktionsraum) eine einmal ermittelte Mindestverweilzeit für die vollständige Reaktion um weniger als 20%, vorzugsweise weniger als 10% überschritten, kann die Bildung von Folge-Reaktionsprodukten wie Isocyanuraten und Carbodiimiden aus den gebildeten aromatischen Diisocyanaten weitgehend vermieden werden.

Bei der praktischen Durchführung des Verfahrens kann eine Abweichung von der mittleren Verweilzeit (Kontaktzeit) durch die notwendige Zeit der Vermischung der Reaktionspartner erfolgen. Solange die Reaktionspartner noch nicht homogen vermischt sind, sind im Reaktor noch unvermischte oder teilvermischte Gasvolumina vorhanden, in denen noch kein oder noch kein vollständiger Kontakt der Reaktionspartner erfolgt ist. Die Vermischung der Reaktionspartner soll daher bevorzugt innerhalb einer Zeit von 0,01 bis 0,3 Sekunden bis zu einem Segregationsgrad von mindestens 10⁻¹ erfolgen. Der Segregationsgrad ist ein Maß für die Unvollständigkeit der Vermischung (siehe z.B. Chem.-Ing.-Techn. 44, (1972), S. 1051ff; Appl.SCi.Res.(The Hague) A3 (1953), S.279).

Die Methoden zur Durchführung kurzer Mischzeiten sind im Prinzip bekannt. Wie oben ausgeführt sind beispielsweise Mischaggregate oder Mischzonen mit bewegten oder statischen Mischorganen oder Düsen geeignet. Bevorzugt sind statische Mischer, wie sie z.B. in EP-A-1 362 847, EP-A-1 526 129 oder EP-A- 1 555 258 beschrieben sind.

Nach Vermischung der Reaktionskomponenten durchströmt das Reaktionsgemisch den Reaktionsraum. Dabei weist bevorzugt weder die Mischzone noch der daran anschließende Reaktionsraum Heizflächen auf, die Anlass zu einer thermischen Belastung mit der Folge von Folgereaktionen wie Isocyanurat- oder Carbodiimid-Bildung Anlass geben können, oder Kühlflächen auf, die Anlass einer Kondensation mit der Folge von Ablagerungen geben können. Die Komponenten werden bevorzugt adiabat umgesetzt. Dabei wird dann die adiabate Temperaturerhöhung im Reaktor allein über die Temperaturen, die Zusammensetzungen und relativen Dosierungen der Eduktströme sowie der Verweilzeit im Reaktor eingestellt.

Die Durchströmung des Reaktionsraums soll bevorzugt in Form einer zu 90% angenäherten Pfropfenströmung erfolgen, so dass alle Volumenteile der Strömung angenähert die gleiche Strömungszeit aufweisen, damit eine möglichst geringe weitere Verbreiterung der Verweilzeitverteilung zwischen den Reaktionspartnern erfolgt. Der Grad der Realisierung der idealen Pfropfenströmung (mit einer mittleren Abweichung von der mittleren Verweilzeit = 0) wird in der Strömungstechnik durch die Bodenstein-Zahl Bo (Fitzer, Techn. Chemie, Springer 1989, S. 288-295) beschrieben. Bevorzugt soll die Bodenstein-Zahl im erfindungsgemäßen Verfahren mindestens 10, bevorzugt größer 100, besonders bevorzugt größer 250 betragen.

Nach der erfolgten Phosgenierungsreaktion im Reaktionsraum wird in Schritt c) das den Reaktionsraum kontinuierlich verlassende Gasgemisch, das bevorzugt wenigstens ein aromatisches Diisocyanat, Phosgen und Chlorwasserstoff umfasst, von dem gebildeten aromatischen Diisocyanat bevorzugt weitestgehend befreit. Dies kann beispielsweise einstufig durch selektive Kondensation in einem inerten Lösungsmittel erfolgen, wie es bereits für andere Gasphasenphosgenierungen empfohlen wurde (EP-A-0 749 958).

Bevorzugt erfolgt die Kondensation jedoch dadurch, dass in das den Reaktionsraum verlassende Gasgemisch ein oder mehrere geeignete Flüssigkeitsströme (Quench-Flüssigkeiten) eingesprüht werden. Dadurch kann, wie in EP-A- 1 403 248 beschrieben, eine rasche Abkühlung des Gasgemisches ohne den Einsatz kalter Flächen durchgeführt werden. Unabhängig von der Art der gewählten Abkühlung wird die Temperatur der Abkühlzone jedoch bevorzugt so gewählt, dass sie einerseits oberhalb der Zersetzungstemperatur der dem aromatischen Diisocyanat entsprechenden Carbamidsäurechloride liegt und andererseits das aromatische Diisocyanat und gegebenenfalls das in dem Amindampfstrom und / oder Phosgenstrom als Verdünnungsmittel mit verwendete Lösungsmittel kondensieren bzw. sich in dem Lösungsmittel lösen, während überschüssiges Phosgen, Chlorwasserstoff und ggf. als Verdünnungsmittel mit verwendetes Inertgas die Kondensations- bzw. Quenchstufe durchlaufen. Zur selektiven Gewinnung des aromatischen Diisocyanats aus dem den Reaktionsraum gasförmig verlassenden Gemisch besonders gut geeignet sind bei einer Temperatur von 80 bis 200°C, vorzugsweise 80 bis 180 °C gehaltene Lösungsmittel wie beispielsweise Chlorbenzol und / oder Dichlorbenzol, oder in diesem Temperaturbereichen gehaltenes aromatisches Diisocyanat oder Mischungen des aromatischen Diisocyanats mit Chlorbenzol und / oder Dichlorbenzol.

Die Erzeugung der für das erfindungsgemäße Verfahren wesentlichen Strömung des gasförmigen Reaktionsgemischs als weitgehende Pfropfenströmung ohne wesentliche Rückvermischung ausgehend von der Mischzone durch den Reaktionsraum wird vorteilhaft durch ein Druckgefälle zwischen den Eduktzuleitungen zur Mischzone einerseits und dem Ausgang aus der Kondensations- bzw. Quenchstufe andererseits sichergestellt. Im Allgemeinen liegt der absolute Druck in den Zuleitungen zu der Mischzone bei 200 bis 3000 mbar und hinter der Kondensations- bzw. Quenchstufe bei 150 bis 2500 mbar. Wesentlich ist hierbei jedoch lediglich die Aufrechterhaltung eines Differenzdrucks zwecks Gewährleistung der genannten gerichteten Strömung.

Das die Kondensations- bzw. Quenchstufe verlassende Gasgemisch wird in Schritt d) in einer nachgeschalteten Gaswäsche mit einer geeigneten Waschflüssigkeit, bevorzugt inerten Lösungsmitteln, z.B. aromatischen Kohlenwasserstoffen ohne oder mit Halogensubstitution, wie z.B. Chlorbenzol oder o-Dichlorbenzol, besonders bevorzugt das ggfs. zur Verdünnung der Edukte und in der Quenche eingesetzte Lösungsmittel, von restlichem Isocyanat befreit, anschließend in an sich bekannter Weise von überschüssigem Phosgen befreit. Dies kann mittels einer Kühlfalle, Absorption in einem inerten Lösungsmittel (z.B. Chlorbenzol oder Dichlorbenzol) oder durch Adsorption und Hydrolyse an Aktivkohle erfolgen. Bevorzugt ist die Absorption in einem inerten Lösungsmittel, wobei die entstandenen Phosgenlösungen bevorzugt direkt oder nach entsprechender Aufbereitung einer Phosgendesorptionseinheit zugeführt werden, so das im Überschuss eingesetzte Phosgen für einen erneuten Einsatz recycliert wird. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die in Schritt d) nach ihrem Einsatz zur Gaswäsche anfallende Waschflüssigkeit wird bevorzugt als Quench-Flüssigkeit zur Abkühlung des aus dem Rohrreaktor austretenden Gasgemisches in Schritt c) eingesetzt.

Die Reindarstellung der aromatischen Diisocyanate erfolgt bevorzugt anschließend durch destillative Aufarbeitung der aus der Kondensations- bzw. Quenchstufe erhaltenen Lösungen bzw. Gemische.

### Beispiele:

### Beispiel 1 (erfindungsgemäß):

Durch Vermischung in einer Düse werden in einem rotationssymetrischen Rohrreaktor ein Gemisch bestehend aus gasförmigen 2,4- und 2,6-Toluylendiamin und Phosgen, die jeweils getrennt auf oberhalb 300°C erhitzt wurden, adiabatisch mit einer Verweilzeit von weniger als 10 Sekunden (und mehr als 0,05 Sekunden) umgesetzt. Das Gasgemisch wird durch eine Kondensationsstufe geleitet und dabei auf eine Gastemperatur von ca. 165°C abgekühlt. Das anfallende Kondensat wird einer Destillationssequenz zugeführt und ergibt reines TDI. Das nicht kondensierte Gasgemisch wird in einer anschließenden Wäsche mit ODB gewaschen und das Nebenprodukt HCl vom überschüssigen Phosgen absorptiv getrennt. Die Waschflüssigkeit wird in dem Kondensationsschritt eingesetzt.

Die Druckdifferenz zwischen dem Druck in der TDA-Zuleitung und am Kopf der Waschstufe beträgt 21 mbar, um eine gerichtete Gasströmung von den Zuleitungen zu erreichen.

Das eingesetzte Gemisch bestehend aus 2,4- und 2,6-Toluylendiamin enthält 0,01 mol-% an keine Ketogruppen enthaltenden, aliphatischen Amine in der Summe pro mol primärer Aminogruppen.

Nach 300 Stunden Versuchszeit beträgt der Differenzdruck 28 mbar und ist damit im Rahmen der Messgenauigkeit großtechnischer Messinstrumente unverändert. Eine Inspektion zeigt keine Ablagerungen von Feststoffen.

### Beispiel 2 (nicht erfindungsgemäß):

Durch Vermischung in einer Düse werden in einem rotationssymetrischen Rohrreaktor ein Gemisch bestehend aus gasförmigen 2,4- und 2,6-Toluylendiamin und Phosgen, die jeweils getrennt auf oberhalb 300°C erhitzt wurden, adiabatisch mit einer Verweilzeit von weniger als 10 Sekunden (und mehr als 0,05 Sekunden) umgesetzt. Das Gasgemisch wird durch eine Kondensationsstufe geleitet und dabei auf eine Gastemperatur von ca. 165°C abgekühlt. Das anfallende Kondensat wird einer Destillationssequenz zugeführt und ergibt reines TDI. Das nicht kondensierte Gasgemisch wird in einer anschließenden Wäsche mit ODB gewaschen und das Nebenprodukt HCl vom überschüssigen Phosgen absorptiv getrennt. Die Waschflüssigkeit wird in dem Kondensationsschritt eingesetzt.

Die Druckdiffrenz zwischen dem Druck in der TDA-Zuleitung und am Austritt der Kondensationsstufe betrug bei Versuchsstart 50 mbar, um eine gerichtete Gasströmung von den Zuleitungen zu erreichen.

Das eingesetzte Gemisch bestehend aus 2,4- und 2,6-Toluylendiamin enthält 0,07 mol-% an keine Ketogruppen enthaltenden, aliphatischen Amine in der Summe pro mol primärer Aminogruppen.

Nach 30 Stunden Versuchszeit steigt der Druck in der gasförmigen TDA Zuleitung deutlich an und der Differenzdruck zwischen TDA-Zuleitung und Austritt der Kondensationsstufe beträgt 3177 mbar. Die Reaktion muss daraufhin abgebrochen werden. Eine Inspektion zeigt sehr starke Ablagerungen von Feststoffen.

## Patentansprüche

1. Verfahren zur Herstellung primärer aromatischer Diisocyanate durch Umsetzung der entsprechenden primären aromatischen Diamine mit Phosgen in der Gasphase innerhalb einer mittleren Verweilzeit von 0,05 bis 15 Sekunden, **dadurch gekennzeichnet, dass**
die eingesetzten primären aromatischen Diamine weniger als 0,05 mol-% an keine Ketogruppen enthaltenden, aliphatischen Amine in der Summe pro mol primärer Aminogruppen enthalten.

2. Verfahren nach Anspruch 1, **dadurch gekennzeichnet, dass** als primäres aromatisches Diamin Toluylendiam in eingesetzt wird.

3. Verfahren nach einem der Ansprüche 1 oder 2 , bei dem die primären aromatischen Diamine vor der Reaktion verdampft und, ggf. verdünnt mit einem Inertgas oder mit den Dämpfen eines inerten Lösungsmittels, auf Temperaturen von 200 bis 600 °C erhitzt werden, wobei die verdampften aromatischen Diamine im wesentlichen keine Tröpfchen an unverdampften aromatischen Diaminen enthalten.

4. Verfahren nach einem der Ansprüche 1 bis 3, **dadurch gekennzeichnet, dass** man
a) die dampfförmigen aromatischen Diamine, gegebenenfalls verdünnt mit einem Inertgas oder mit den Dämpfen eines inerten Lösungsmittels, und Phosgen getrennt auf Temperaturen von 200 bis 600 °C erhitzt und kontinuierlich vermischt, wobei ein Reaktionsgemisch erhalten wird,
b) das in Schritt a) erzeugte Reaktionsgemisch unter Vermeidung von Rückvermischung kontinuierlich durch einen Reaktionsraum leitet und es darin innerhalb einer mittleren Verweilzeit von 0,05 bis 15 Sekunden umsetzt, wobei ein Gasgemisch erhalten wird,
c) das aus dem Reaktionsraum austretende Gasgemisch zur Kondensation der gebildeten aromatischen Diisocyanate abkühlt, wobei die Temperatur oberhalb der Zersetzungs-temperatur der den umgesetzten aromatischen Diaminen entsprechenden Carbamidsäurechloriden gehalten wird, und
d) nicht kondensiertes aromatisches Diisocyanat aus dem Gasgemisch durch Waschen mit einer Waschflüssigkeit abtrennt.

5. Verfahren nach Anspruch 4, **dadurch gekennzeichnet, dass** die Umsetzung in Schritt b) adiabat erfolgt.

6. Verfahren nach einem der Ansprüche 4 oder 5, **dadurch gekennzeichnet, dass** man in Schritt b) das in Schritt a) erzeugte Reaktionsgemisch durch einen Reaktionsraum leitet, der eine rotationssymmetrische Geometrie mit einer in Strömungsrichtung des Reaktionsgemischs konstanten oder steigenden durchströmten Querschnittsfläche aufweist.

7. Verfahren nach einem der Ansprüche 4 oder 5, **dadurch gekennzeichnet, dass** man in Schritt b) das in Schritt a) erzeugte Reaktionsgemisch durch einen Reaktionsraum leitet, der eine rotationssymmetrische Geometrie mit einer in Strömungsrichtung des Reaktionsgemischs konstanten und/oder zunehmenden durchströmten Querschnittsfläche aufweist.

8. Verfahren nach einem der Ansprüche 4 bis 7, **dadurch gekennzeichnet, dass** das aus dem Reaktionsraum austretende Gasgemisch wenigstens ein aromatisches Diisocyanat, Phosgen und Chlorwasserstoff enthält und in Schritt c) durch Einsprühen wenigstens eines Flüssigkeitsstroms in das Gasgemisch abgekühlt wird.

9. Verfahren nach Anspruch 8, **dadurch gekennzeichnet, dass** zur Abkühlung des aus dem Reaktionsraum austretenden Gasgemisches in Schritt c) zumindest teilweise die in Schritt d) nach ihrem Einsatz zur Gaswäsche anfallenden Waschflüssigkeit eingesetzt wird.

## Claims

1. Process for preparing primary aromatic diisocyanates by reacting the corresponding primary aromatic diamines with phosgene in the gas phase within a mean residence time of 0.05 to 15 seconds, **characterized in that** the primary aromatic diamines used contain less than 0.05 mol% of aliphatic amines containing no keto groups, in total, per mole of primary amino groups.

2. Process according to Claim 1, **characterized in that** tolylenediamine is used as primary aromatic diamine.

3. Process according to either of Claims 1 and 2, in which the primary aromatic diamines are evaporated before the reaction and, optionally diluted with an inert gas or with the vapours of an inert solvent, are heated to temperatures of 200 to 600°C, the evaporated aromatic diamines comprising substantially no droplets of unevaporated aromatic diamines.

4. Process according to any of Claims 1 to 3, **characterized in that**
a) the vaporous aromatic diamines, optionally diluted with an inert gas or with the vapours of an inert solvent, and phosgene are heated separately to temperatures of 200 to 600°C and mixed continuously, to give a reaction mixture,
b) the reaction mixture produced in step a) is passed continuously, with avoidance of backmixing, through a reaction chamber, in which it is reacted within a mean residence time of 0.05 to 15 seconds, to give a gas mixture,
c) the gas mixture emerging from the reaction chamber is cooled for the condensation of the aromatic diisocyanates formed, the temperature being held above the decomposition temperature of the carbamoyl chlorides corresponding to the aromatic diamines reacted, and
d) uncondensed aromatic diisocyanate is removed from the gas mixture by washing with a wash liquid.

5. Process according to Claim 4, **characterized in that** the reaction in step b) takes place adiabatically.

6. Process according to either of Claims 4 and 5, **characterized in that**, in step b), the reaction mixture produced in step a) is passed through a reaction chamber which has a rotationally symmetrical geometry with a flow-traversed cross-sectional area which is constant or increasing in the flow direction of the reaction mixture.

7. Process according to either of Claims 4 and 5, **characterized in that**, in step b), the reaction mixture produced in step a) is passed through a reaction chamber which has a rotationally symmetrical geometry with a flow-traversed cross-sectional area which is constant and/or increasing in the flow direction of the reaction mixture.

8. Process according to any of Claims 4 to 7, **characterized in that** the gas mixture emerging from the reaction chamber comprises at least one aromatic diisocyanate, phosgene and hydrogen chloride and is cooled in step c) by sprayed introduction of at least one liquid stream into the gas mixture.

9. Process according to Claim 8, **characterized in that** the gas mixture emerging from the reaction chamber is cooled in step c) using at least partly the wash liquid obtained in step d) after it has been used for gas washing.

## Revendications

1. Procédé de préparation de diisocyanates aromatiques primaires par conversion des diamines aromatiques primaires correspondantes avec du phosgène en phase gazeuse à l'intérieur d'un temps de séjour moyen de 0,05 à 15 secondes, **caractérisé en ce que** les diamines aromatiques primaires utilisées contiennent moins de 0,05 % molaires d'amines aliphatiques ne contenant aucun groupe cétone, dans la somme des groupes aminés primaires par mole.

2. Procédé selon la revendication 1, **caractérisé en ce que** l'on utilise comme diamine aromatique primaire la toluylène diamine.

3. Procédé selon l'une quelconque des revendications 1 ou 2, dans lequel les diamines aromatiques primaires sont vaporisées avant la réaction et, éventuellement en étant diluées avec un gaz inerte ou avec les vapeurs d'un solvant inerte, chauffées à des températures de 200 à 600°C, dans lequel les diamines aromatiques vaporisées ne contiennent essentiellement aucune gouttelette de diamines aromatiques non vaporisées.

4. Procédé selon l'une quelconque des revendications 1 à 3, **caractérisé en ce que**
a) on chauffe séparément à des températures de 200 à 600°C les diamines aromatiques vaporisées, diluées éventuellement avec un gaz inerte ou avec les vapeurs d'un solvant inerte, et le phosgène et on les mélange en continu, et on obtient ainsi un mélange de réaction,
b) on conduit le mélange de réaction produit à l'étape a) en continu à travers une chambre de réaction, en évitant un remélange, et on le convertit dans celle-ci à l'intérieur d'un temps de séjour moyen de 0,05 à 15 secondes, et on obtient ainsi un mélange gazeux,
c) on refroidit le mélange gazeux sortant de la chambre de réaction pour la condensation des diisocyanates aromatiques formés, la température étant maintenue au-dessus de la température de décomposition des chlorures d'acide carbamidique correspondant aux diamines aromatiques converties, et
d) on sépare le diisocyanate aromatique non condensé hors du mélange par lavage avec un liquide de lavage.

5. Procédé selon la revendication 4, **caractérisé en ce que** l'on effectue la conversion à l'étape b) de manière adiabatique.

6. Procédé selon l'une quelconque des revendications 4 ou 5, **caractérisé en ce que**, à l'étape b), on conduit le mélange de réaction produit à l'étape a) à travers une chambre de réaction, qui présente une géométrie symétrique de révolution avec une aire de section transversale traversée constante ou croissante dans le sens d'écoulement du mélange de réaction.

7. Procédé selon l'une quelconque des revendications 4 ou 5, **caractérisé en ce que**, à l'étape b), on conduit le mélange de réaction produit à l'étape a) à travers une chambre de réaction, qui présente une géométrie symétrique de révolution avec une aire de section transversale traversée constante et/ou croissante dans le sens d'écoulement du mélange de réaction.

8. Procédé selon l'une quelconque des revendications 4 à 7, **caractérisé en ce que** le mélange gazeux sortant de la chambre de réaction contient au moins un diisocyanate aromatique, du phosgène et du gaz chlorhydrique et est refroidi à l'étape c) par pulvérisation d'au moins un courant de liquide dans le mélange gazeux.

9. Procédé selon la revendication 8, **caractérisé en ce que** l'on utilise, pour le refroidissement du mélange gazeux sortant de la chambre de réaction à l'étape c), au moins partiellement le liquide de lavage recueilli à l'étape d) après son utilisation pour le lavage du gaz.
